# EUROPÄISCHE PATENTSCHRIFT

(11) **EP 0 578 066 B1**
(45) Veröffentlichungstag und Bekanntmachung des Hinweises auf die Patenterteilung: **06.05.1998**
(21) Anmeldenummer: 93110108.3
(22) Anmeldetag: 24.06.1993
(51) Int. Cl.: G01N 33/532, C07J 41/00, C07J 19/00, C12Q 1/68

(54) **Fotochemische Markierung von Nukleinsäuren mit Digoxigenin-Reagenzien und ihre Verwendung in Gensondentestsystemen**
Photochemical labelling of nucleic acids with digoxigenin-reagents and their use in gene probe systems
Marquage photochimique d'acides nucléiques avec des réactifs de digoxigénine et leur utilisation dans des systèmes de sondes génétiques

(30) Priorität: 07.07.1992 DE 4222254
(43) Veröffentlichungstag der Anmeldung: 12.01.1994
(73) Patentinhaber: BAYER AG, 51368 Leverkusen (DE)
(72) Erfinder: Löbberding, Antonius, Dr., D-42113 Wuppertal (DE); Mikhail, Gamal K., Dr., D-51519 Odenthal (DE); Springer, Wolfgang, Dr., D-42113 Wuppertal (DE)

(56) Entgegenhaltungen:
- EP-A- 0 187 332
- EP-A- 0 324 474
- ANNALS OF THE NEW YORK ACADEMY OF SCIENCES Bd. 346, 1980, NEW YORK NY US Seiten 355 - 367 PILL-SOON SONG AND CHING-NAN OU 'Labeling of Nucleic Acids with Psoralens'
- BIOLOGICAL CHEMISTRY HOPPE-SEYLER Bd. 371, Nr. 10, Oktober 1990, BERLIN DE Seiten 917 - 927 CHRISTOPH KESSLER, HANS-JOACHIM H LTKE, RUDOLF SEIBL, JOSEF BURG AND KLAUS M]HLEGGER 'Non-radioactive Labeling and Detection of Nucleic Acids'
- ANALYTICAL BIOCHEMISTRY Bd. 192, Nr. 1, Januar 1991, NEW YORK US Seiten 222 - 231 GUDRUN G. SCHMITZ, THOMAS WALTER, RUDOLF SEIBL, AND CHRISTOPH KESSLER 'Nonradioactive Labeling of Oligonucleotides in Vitro with the Hapten Digoxigenin by Tailing with Terminal Transferase'

## Beschreibung

Die Gensondendiagnostik ist eine Methode zum sequenzspezifischen Nachweis von DNA/RNA-Sequenzen. Sie basiert auf der Hybridisierung der Gensondensequenz mit komplementären Sequenzbereichen der nachzuweisenden DNA/RNA (J.A. Matthews, L.J. Kricka, Analytical Biochemistry **169**, 1-25 (1988): U. Landegren, R. Kaiser, C.T.Caskey, L.Hood, Science **242**, 229 (1988)).

Die Gensondendiagnostik ermöglicht den Nachweis von Infektionskrankheiten und genetischen Defekten. Voraussetzung für eine breite Anwendung der Gensondendiagnostik ist eine ausreichende Nachweisempfindlichkeit, eine einfache Durchführbarkeit sowie die Vermeidung von Radioaktivität.

Eine Variante der Gensondendiagnostik verläuft über die direkte fotochemische Markierung der nachzuweisenden DNA/RNA; anschließend erfolgt eine Hybridisierung an Gensonden mit komplementären Nukleinsäuresequenzen (N. Dattagupta, P.M.M. Rae, E.D. Huguenel, E. Carlson, A. Lyga, J.S. Shapiro, J.P. Albarella, Analytical Biochemistry **177**, 85 (1989); J.P. Albarella, R.L. Minegar, W.L. Patterson, N. Dattagupta, E. Carlson, Nucleic Acids Research **17**, 4293 (1989)).

Furocoumarine, die über geeignete Spacermoleküle mit Biotin verknüpft sind, erwiesen sich als gut geeignet zur Fotobiotinylierung von Nukleinsäuren. Nach der Hybridisierung an eine Gensonde mit einer komplementären Nukleinsäuresequenz und einem Trennschritt erfolgt die Detektion, beispielsweise durch Zufügen eines Komplexes aus Antibiotin-Antikörper bzw. Avidin oder Streptavidin mit alkalischer Phosphatase. Zum Nachweis wird in einem weiteren Schritt eine Farbreaktion durchgeführt, die durch alkalische Phosphatase ausgelöst wird (J.J. Leary, D.J. Brigati, D.C. Ward, Proc. Natl. Acad. Sci. USA **80**, 4045-4049 (1983)).

Ein Nachteil des Nachweissystems über Biotin ist die weite Verbreitung von Biotin in biologischen Systemen. Dieser Nachteil wird beispielsweise bei einer Verwendung von Digoxigenin anstatt von Biotin vermieden. Die Nachweisreaktion erfolgt hier über Digoxigenin-Antikörper.

Bisherige Methoden zur Markierung von Nukleinsäuren mit Digoxigenin verlaufen enzymatisch oder fotochemisch (G.G. Schmitz, T. Walter, R. Seibl, C. Kessler, Analytical Biochemistry **192**, 222-231 (1991); C. Kessler, H.-J. Höltke, R. Seibl, J. Burg, K. Mühlegger, Biol. Chem. Hoppe-Seyler **371,** 917-927 (1990)). Im Falle der Fotomarkierung mit dem Fotodigoxigenin-Reagenz der Fa Boehringer (EP-324 474-A) wurde eine irreversible Denaturierung der nachzuweisenden Nukleinsäuren beobachtet.

Überraschenderweise wurde bei Fotoreaktionen mit Digoxigeninreagenzien, die über einen geeigneten Spacer mit Furocoumarinen verknüpft sind, keine Denaturierung der Nukleinsäuren beobachtet

In der vorliegenden Erfindung wird die Synthese von Digoxigenin-Derivaten, die über geeignete Spacer mit Furocoumarinen verknüpft sind beschrieben, sowie ihre Verwendung zur Markierung von Nukleinsäuren untersucht.

Erfindungsgemäß wird ein Markierungsreagenz der allgemeinen Formel:

**Dig - S - Fu**

synthetisiert, wobei
- **Dig** =: Digoxigeninderivat als Hapten,
- **S** =: ein Spacer Molekül, und
- **Fu** =: ein Furocumarinderivat als fotochemisch verknüpfbare Struktur.

Das Digoxigeninderivat (**Dig**) ist ein chemisch modifiziertes Derivat des Steroids wobei die C-3-Hydroxygruppe mit einem chemisch verknüpfbaren Substituenten modifiziert ist. Dies kann eine Carboxyl-, Thio-, Amino- oder Hydroxygruppierung sein, bzw. aktivierte Form davon

Geeignete Furocumarine und Spacer sind in der EP 187 332 beschrieben.

Der Spacer ist ein Polyalkylamin, Polyethylenglykol oder eine Kombination davon.

Polyalkylamine haben folgende allgemeine Formel: 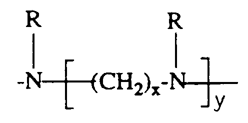 wobei:
- R: für H, C₁-C₇-Alkyl, Aryl (wie z.B. Phenyl, Naphthyl oder Anthracyl), Hydroxy oder C₁-C₇-Alkoxy;
- x: für eine Zahl zwischen 2 und 7;
- y: für eine Zahl zwischen 3 und 10 steht.

R kann in den unterschiedlich möglichen, o.g. Varianten vorkommen, d.h., R muß nicht identisch sein bei jeder Wiederholung der -(CH₂)ₓ-N-R-Einheit im Spacer. Das gleiche gilt für x, d.h., x muß nicht die gleiche Zahl sein bei jeder Wiederholung der -(CH₂)ₓ-Einheit im Spacer.

Bevorzugt sind unabhängig voneinander die R's = H, C₁-C₄-Alkyl (z.B. Methyl, Ethyl, n-Propyl, i-Propyl, n-Butyl, i-Butyl, tert.-Butyl); x = 2, 3, 4 oder 5; und y = 3, 4, 5 oder 6.

Besonders bevorzugt sind N⁴,N⁹-Dimethylspermin-Derivate der Formel

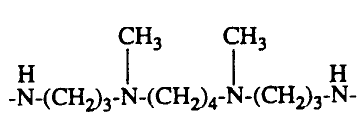

Polyethylenglykole haben folgende allgemeine Formel: 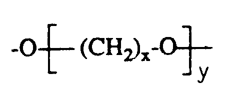 wobei
- x =: eine Zahl zwischen 2 und 7 und
- y =: eine Zahl zwischen 3 und 10 sind.

Bevorzugt sind Polyethylenglykole mit x = 2, 3, 4 oder 5; y = 3, 4, 5 oder 6.

Besonders bevorzugt sind Polyethylenglykole mit x = 2 und y = 4, 5 oder 6.

Spacer-Moleküle mit kombinierten Amin/Glykolstrukturen haben folgende allgemeine Formel: 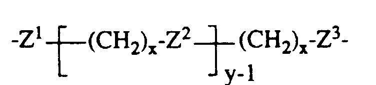 wobei
- Z¹, Z² und Z³: unabhängig voneinander für O oder NR stehen,
- R: für H, C₁-C₇-Alkyl, Aryl (wie z.B. Phenyl, Naphthyl oder Anthracyl), Hydroxy oder C₁-C₇-Alkoxy;
- x: für eine Zahl zwischen 2 und 7;
- y: für eine Zahl zwischen 3 und 10 steht.

Bevorzugt sind Spacer-Strukturen mit Z² = O und Z¹, Z³ = NR, wobei R =H, C₁-C₄-Alkyl (z.B. Methyl, Ethyl, n-Propyl, i-Propyl, n-Butyl, i-Butyl, tert.-Butyl); x = 2, 3, 4 oder 5; und Y = 3, 4, 5 oder 6.

Besonders bevorzugt sind Strukturen mit Z² = O, Z¹, Z³ = NR, wobei R = H, Methyl, Ethyl; x = 2; und y = 4.

Geeignete fotochemisch verknüpfbare Strukturen sind insbesondere Furocumarine, wie z.B. Angelicin (Isopsoralen) bzw. Psoralene sowie Derivate davon, die mit Nucleinsäuren fotochemisch reagieren.

Angelicin-Derivate haben folgende allgemeine Formel: 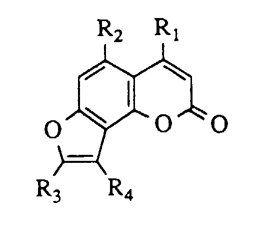 wobei
- R₁, R₂ und R₃: unabhängig voneinander für H oder C₁-C₇-Alkyl, und
- R₄: für H, C₁-C₇-Alkyl oder niedriges Alkyl mit Hydroxy, C₁-C₇-Alkoxy-, Amino-, Halo- oder N-Phthalimidosubstituenten steht,

Besonders bevorzugt sind Angelicin-Derivate die folgende R₁-R₄-Gruppierungen enthalten:

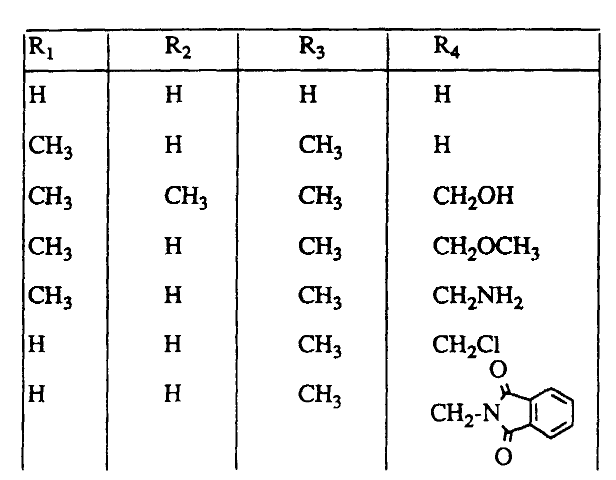

Andere Verbindungen mit unterschiedlichen R's können auch nach literaturbekannten Verfahren synthetisiert werden.

Geeignete Psoralene haben folgende allgemeine Formel: 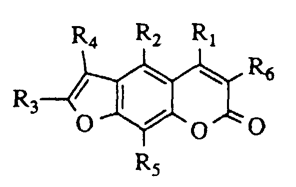 wobei
- R₁, R₃ und R₆: unabhängig voneinander für H oder C₁-C₇-Alkyl,
- R₄: für H, C₁-C₇-Alkyl oder C₁-C₇-Alkyl mit Hydroxy-, C₁-C₇-Alkoxy-, Amino-, Halo- oder N-Phthalimidosubstituenten,
- R₂ und R₅: unabhängig voneinander für H, Hydroxy, Carboxy, Carbo-C₁-C₇-alkoxy oder C₁-C₇-Alkoxy steht.

Angelicin-Derivate sind vorteilhaft im Vergleich zu Psoralenen aufgrund der Monoaddukt-Bildung.

Die Reihenfolge der Bindung des Digoxigenins, des Spacers und des Furocumarin ist beliebig. Es ist also unter anderem möglich, zuerst Digoxigenin **Dig** mit dem Spacer **S** zu verknüpfen und anschließend mit dem Furocumarin **Fu** umzusetzen. Umgekehrt kann **Fu-S** zuerst aufgebaut und dann mit **Dig** umgesetzt werden.

Die Verknüpfung der Bausteine erfolgt in an sich bekannter Weise.

### Beispiel 1

Herstellung von Aminohexaethylenglykolangelicin (Amino-PEG-angelicin ( 1 ) 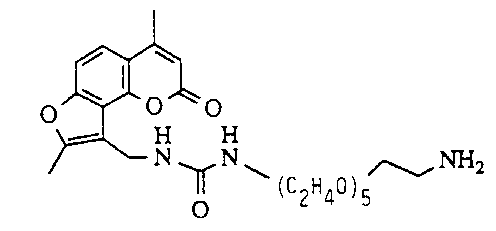 4,87 g (20 mmol) 4-Aminomethyl4,5'-dimethylangelicin werden in 25 ml DMF gelöst und bei Raumtemperatur mit 3,24 g (20 mmol) Carbonyldiimidazol umgesetzt. Eine vollständige Umsetzung (lt. DC) wurde nach 6 Stunden Rühren unter Stickstoff festgestellt. Die Lösung wird zu einer Lösung aus 16,85 g (60 mmol) 1,17-Di-amino-3,6,9,12,15-pentaoxaheptadecan in 40 ml DMF bei 80°C langsam zugetropft und weiter bei 70°C für 12 Stunden gerührt. Nach dem Abkühlen wird die Lösung im Vakuum eingeengt und auf Kieselgel chromatographiert (Laufmittel: Chloroform, Methanol, Ammoniak 90:10:1, Rf = 0,28). Man erhält 7,1 g (65 % der Theorie) eines leichtgelben Öls.

### Beispiel 2

Herstellung von N¹-(Angeliciamido)-N⁴,N⁹-dimethylspermin (2) 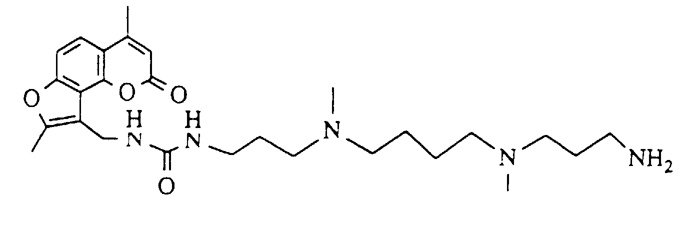 4,87 g (20 mmol) 4-Aminomethyl-4,5'-dimethylangelicin werden mit Carbonyldiimidazol analog wie im Beispiel 1 aktiviert. Die entstandene Lösung wird zu einer Lösung aus 13,8 g (60 mmol) N⁴,N⁹ Dimethylspermin in 40 ml DMF analog Beispiel 1 zugetropft Nach dem Abkühlen wird die Lösung in Vakuum eingeengt und der Rückstand auf Kieselgel chromatographiert (Laufmittel Chloroform/-Methanol/Ammoniak 30:5:11, Rf = 0,11). Man erhält 7,1 g (71 % der Theorie) eines gelben Öls.

### Beispiel 3

Herstellung von Digoxigenin PEG-Angelicin (Dig-PA (3)) 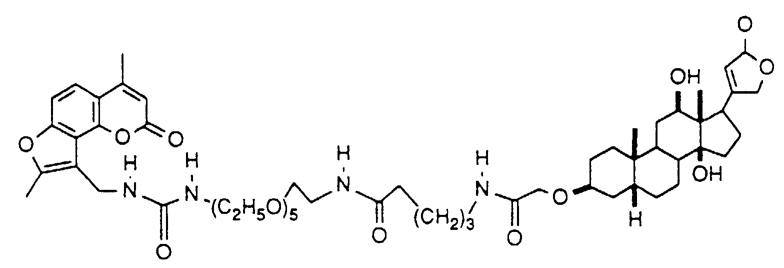 13,7 mg (0,03 mmol) Amino-PEG-angelicin 1 werden in 2 ml Chloroform (für Spektroskopie). Eine Lösung von 18,9 mg (0,03 mmol) Digoxigenin-3-O-methylcarbonyl-ε-aminocapronsäure-N-hydroxysuccinimidester (Dig-NHS) in 2 ml Chloroform wird zugetropft. Nach 24 Stunden Rühren bei Raumtemperatur ist die Reaktion beendet (lt. DC). Die Lösung wird im Vakuum eingeengt und der Rückstand auf Kieselgel chromatographiert (Laufmittel Chloroform/Methanol/Ammoniak /Ammoniak 90:10:1, Rf = 0,45). Man erhält 21 mg (77 % der Theorie) eines leichtgelben Feststoffs mit Schmelzpunkt 63 bis 65°C.

### Beispiel 4

Herstellung von Digoxigenin-Spermin-Angelicin (Dig-SpA, (4)) 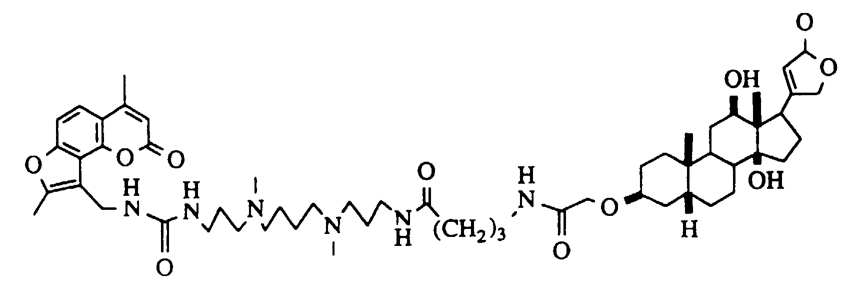 15 mg (0,03 mmol) der in Beispiel 2 beschriebenen Verbindung 2 werden analog zu Beispiel 3 mit 19,8 mg (0,03 mmol) Digoxigenin-3-O-methylcarbonyl-ε-aminocapronsäure-N-hydroxy-succinimidester(Dig-NHS)umgesetzt.

Nach dem Aufarbeiten und Säulenchromatographie auf Kieselgel (Laufmittel Chloroform/Methanol/Ammoniak 30:5:1, Rf = 0,25) erhält man 24 mg (77 % der Theorie) eines schwach gelblichen Feststoffs des Schmelzpunkts 106 bis 109°C.

### Beispiel 5

### Fotoreaktion von Hairpin-Oligonukleotiden mit DigPA

50 µg des Hairpin-Oligonukleotids werden in 100 µl Tris-HCl-Puffer aufgenommen. Die Lösung wird im Wasserbad für 15 Minuten bei 50°C belassen. Zum langsamen Abkühlen auf Raumtemperatur wird die Probe aus dem Wasserbad genommen. Anschließend werden weitere 400 µl Wasser hinzugefügt.

Zur Fotoreaktion werden 15 µg des hybridisierten Hairpin-Oligonukleotids mit einem 20fachen molaren Überschuß an DigPA versetzt. Die Lösung wird im Anschluß unter einer UV-Lampe bei 366 nm oder 312 nm in einem Eppendorfgefäß in einem Eisbad belichtet. Die Fotoreaktion wird per HPLC verfolgt. Innerhalb von 15 Minuten war die Fotoreaktion abgeschlossen.

### Beispiel 6

### Fotomarkierung mit DigPA

Zur Fotomarkierung mit DigPA wurden 2 bis 5 µg DNA in 20µl TE-Puffer mit 50µl 1 M Natriumtetraboratpuffer pH 8,3 und 50µl DigPA (2 µg/µl) versetzt und mit bidest. H₂O auf 500 µl aufgefüllt. Der Ansatz wurde 10 Minuten bei 312 nm mit einem UV-Transilluminator bestrahlt, die Proben dabei auf Eis gelagert.

Die fotomarkierte DNA wurde anschließend mit 1/10 Volumen 3 M Natriumacetat pH 5,8 und 1 Volumen Isopropanol bei Raumtemperatur gefällt und 5 Minuten stehen gelassen. Anschließend wurde die DNA bei 10 000 UpM in einer Eppendorf-Zentrifuge abzentrifugiert, der Überstand abdekantiert und der DNA-Niederschlag mit 70 %igem Ethanol gewaschen. Nach der Trocknung der Proben wurde die fotomarkierte DNA in TE aufgenommen. Die Fotomarkierung der DNA mit Digoxigenin wurde anschließend durch Agarosegelelektrophorese und Dot Blot Assays mit Digoxigenin-Antikörper-Konjugaten überprüft.

In der Gelelektrophorese wandert die fotomarkierte DNA langsamer als die nichtmarkierte DNA, was in einem leichten Shift nach oben bei der fotomarkierten DNA-Bande festzustellen ist. Denaturierte DNA in den Geltaschen wurde bei dieser Methode nicht beobachtet.

Die Markierung mit Digoxigenin wurde auch immunologisch mit einem Kit der Firma Boehringer, Mannheim überprüft. Dazu wurde die fotomarkierte DNA in verschiedenen Verdünnungen auf Nitrozellulosemembranen aufgetragen und bei 80°C im Vakuum fixiert. Anschließend wurde die Membran 1 Minute in Puffer 1 (100 mM Tris/HCl, 150 mM NaCl pH 7,5) gewaschen. Dann wurde das Filter 30 Minuten mit 100 ml Puffer 2 (0,5 % Blocking Reagenz in Puffer 1) inkubiert. Anschließend erneut in Puffer 1 gewaschen. Antikörperkonjugat (Antidigoxigenin-Antikörper gekoppelt mit alk. Phosphatase) wurde 1:5 000 auf 150 mU/ml in Puffer 1 verdünnt und das Filter 30 Minuten mit 20 ml der verdünnten Antikörperlösung inkubiert Nichtgebundener Antikörper wurde dann durch 2 x 15 minütiges Waschen mit 100 ml Puffer 1 entfernt. Anschließend wurde die Membran 2 Minuten mit 20 ml Puffer 3 (100 mM Tris/HCl, 100 mM NaCl, 50 mM MgCl₂ pH 9,5) bei Raumtemperatur äquilibriert. Das Filter wurde in einer Plastiktasche mit 10 ml Farblösung (45 µl Nitroblautetrazoliumlösung, 35 µl Bromchlorindolylphosphat in 10 ml Puffer 3) in der Dunkelheit inkubiert. Nach der Entwicklung der Farbflecken wurde die Membran 5 Minuten mit 50 ml Puffer 4 (10 mM Tris/HCl, 1 mM EDTA pH 8) gewaschen um die Farbreaktion zu stoppen.

### Beispiel 7

### Fotomarkierung mit Fotodigoxigenin

Beim Fotodigoxigenin reagiert die Azidophenylgruppe, die über einen hydrophilen Spacer an Digoxigenin gekoppelt ist, bei Belichtung mit Quecksilberdampf (350 bis 700 nm) unspezifisch mit einer Vielzahl von Verbindungen wie z.B. Proteinen und Nukleinsäuren.

Nukleinsäuren wurden nach der Methode von Boehringer mit Fotodigoxigenin markiert 10 µg Fotodigoxigenin-Lösung (10 mg/ml in Dimethylformamid) wurden zu 10 µg DNA gegeben und mit bidest H₂O auf 40 µl aufgefüllt. Das offene Röhrchen wurde in Eiswasser im Abstand von 10 cm unter einer Philipps HPLR 400 W-Lampe plaziert und 15 Minuten bestrahlt. 60 µl Tris/HCl 100 mmol/l pH 9, EDTA 1 mmol/l wurden zugegeben und anschließend 15 µl NaCl 5 mol/l. Es wurde dann zweimal mit 100 µl 2-Butanol extrahiert und die DNA mit 10 µl LiCl 4 mol/l und 100 µl vorgekühltem Ethanol ausgefällt. Nach 40 Minuten bei -70°C wurde bei 12 000 g zentrifugiert und das Pellet mit kaltem 70 %igem Ethanol gewaschen, im Vakuum getrocknet und dann in 40 µl Tris/HCl 10 mmol/l, EDTA 1 mmol/l pH 8 gelöst.

Die Fotomarkierung wurde gelelektrophoretisch und immunologisch mit einem Digoxigenin-Antikörperkonjugat überprüft.

In der Agarosegelelektrophorese wurde festgestellt, daß ein Großteil der fotodigoxigenierten DNA denaturiert in den Geltaschen vorliegt, während mit der in Beispiel 6 beschriebenen DigPA-Methode nur eine Bande der fotomarkierten DNA zu sehen ist und keine denaturierte DNA in den Geltaschen beobachtet wird.

Die immunologische Überprüfung der Fotodigoxigenierung wurde wie im Beispiel 6 beschrieben durchgeführt.

In Dot Blot Tests mit fixierter fotodigoxigenierter DNA wurde darüber hinaus festgestellt, daß die Markierungseffizienz gegenüber dem im Beispiel 6 aufgeführten sehr schonenden DigPA-Verfahren deutlich schlechter ist.

## Patentansprüche

1. Markierungsreagenz der allgemeinen Formel:
**Dig - S - Fu**
wobei
**Dig** = ein Digoxigeninderivat
**S** = ein Spacer Molekül, und
**Fu** = ein Furocumarinderivat ist.

2. Markierungsreagenz gemäß Anspruch 1, wobei das Digoxigeninderivat (**Dig**) ein chemisch modifiziertes Derivat des Steroids ist, worin die C-3-Hydroxygruppe mit einem chemisch verknüpfbaren Substituenten modifiziert ist.

3. Markierungsreagenz gemäß Anspruch 1, wobei der Spacer ein Polyalkylamin, Polyethylenglykol oder eine Kombination davon ist.

4. Markierungsreagenz gemäß Anspruch 1, worin Fu ein Angelicin-Derivat der folgenden allgemeinen Formel ist: 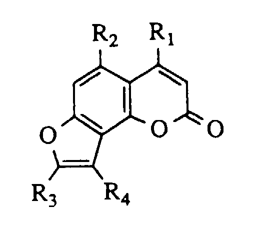 wobei
R₁, R₂ und R₃ unabhängig voneinander für H oder C₁-C₇-Alkyl, und
R₄ für H, C₁-C₇-Alkyl oder niedriges Alkyl mit Hydroxy, C₁-C₇-Alkoxy-, Amino-, Halo- oder N-Phthalimidosubstituiernten steht.

5. Markierungsreagenz gemäß Anspruch 1, worin Fu ein Psoralen der folgenden allgemeinen Formel ist: 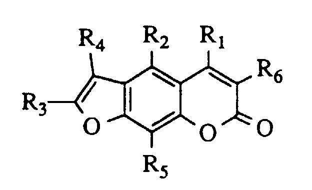 wobei
R₁, R₃ und R₆ unabhängig voneinander für H oder C₁-C₇-Alkyl,
R₄ für H, C₁-C₇-Alkyl oder C₁-C₇-Alkyl mit Hydroxy-, C₁-C₇-Alkoxy-, Amino-, Halo- oder N-Phthalimidosubstituenten,
R₂ und R₅ unabhängig voneinander für H, Hydroxy, Carboxy, Carbo-C₁-C₇-alkoxy oder C₁-C₇-Alkoxy steht.

## Claims

1. Labelling reagent of the general formula:
**Dig - S - Fu**
where
**Dig** is = a digoxigenin derivative
**S** is = a spacer molecule, and
**Fu** is = a furocoumarin derivative.

2. Labelling reagent according to Claim 1, where the digoxigenin derivative (**Dig**) is a chemically modified derivative of the steroid in which the C-3 hydroxyl group is modified with a chemically linkable substituent.

3. Labelling reagent according to Claim 1, where the spacer is a polyalkylamine, polyethylene glycol or a combination thereof.

4. Labelling reagent according to Claim 1, in which **Fu** is an angelicin derivative of the following general formula: 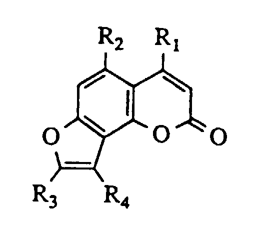
R₁, R₂ and R₃, independently of each other, represent H or C₁-C₇-alkyl, and
R₄ represents H, C₁-C₇-alkyl or lower alkyl having hydroxyl, C₁-C₇-alkoxy, amino, halo or N-phthalimido substituents.

5. Labelling reagent according to Claim 1, in which **Fu** is a psoralen of the following general formula: 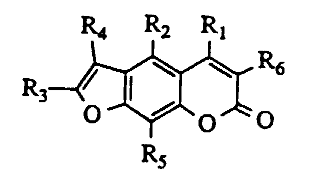 where
R₁, R₃ and R₆, independently of each other, represent H or C₁-C₇-alkyl,
R₄ represents H, C₁-C₇-alkyl or C₁-C₇-alkyl having hydroxyl, C₁-C₇-alkoxy, amino, halo or N-phthalimido substituents,
R₂ and R₅, independently of each other, represent H, hydroxyl, carboxyl, carbo-C₁-C₇-alkoxy or C₁-C₇-alkoxy.

## Revendications

1. Réactif de marqùage de formulé générale
Dig - S - Fu
dans laquelle
Dig désigne un dérivé de digoxigénine
S est une molécule d'espaceur, et
Fu est un dérivé de furocoumarine.

2. Réactif de marquage suivant la revendication 1, dans lequel le dérivé de digoxigénine (Dig) est un dérivé du stéroïde modifié chimiquement, dans lequel le groupe hydroxy de l'atome C-3 est modifié avec un substituant pouvant être lié chimiquement.

3. Réactif de marquage suivant la revendication 1, dans lequel l'espaceur est une polyalkylamine, un polyéthylène-glycol ou une combinaison des deux.

4. Réactif de marquage suivant la revendication 1, dans lequel Fu est un dérivé d'angélicine de formule générale suivante : 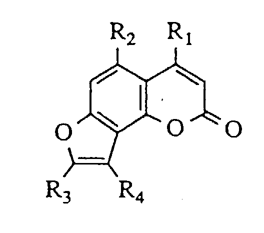 dans laquelle
R₁, R₂ et R₃ représentent, indépendamment les uns des autres, H ou un groupe alkyle en C₁ à C₇, et
R₄ représente H, un groupe alkyle en C₁ à C₇ ou un groupe alkyle inférieur porteur de substituants hydroxy, alkoxy en C₁ à C₇, amino, halogéno ou N-phtalimido.

5. Réactif de marquage suivant la revendication 1, dans lequel Fu est un psoralène de formule générale suivante : 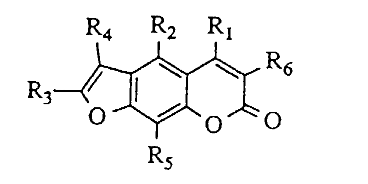 dans laquelle
R₁, R₃ et R₆ représentent, indépendamment les uns des autres, H ou un groupe alkyle en C₁ à C₇,
R₄ représente H, un groupe alkyle en C₁ à C₇ ou un groupe alkyle en C₁ à C₇ portant des substituants hydroxy, alkoxy en C₁ à C₇, amino, halogéno ou N-phtalimido,
R₂ et R₅ représentent, indépendamment l'un de l'autre, H, un groupe hydroxy, carboxy, carbo-(alkoxy en C₁ à C₇) ou alkoxy en C₁ à C₇.
